# EUROPÄISCHE PATENTANMELDUNG

(11) **EP 2 638 885 A1**
(43) Veröffentlichungstag der Anmeldung: **18.09.2013**
(21) Anmeldenummer: 12159260.4
(22) Anmeldetag: 13.03.2012
(51) Int. Cl.: A61F 5/56

(54) **Vorrichtung zur Unterdrückung von Schnarchgeräuschen**

(71) Anmelder: Jensch, Ingolf, 82024 Taufkirchen (DE)
(72) Erfinder: Jensch, Ingolf, 82024 Taufkirchen (DE)
(74) Vertreter: Thun, Clemens

(57) **Zusammenfassung**

Eine Anordnung (1) zur Unterdrückung oder Reduzierung von Bewegungen des menschlichen Unterkiefers im Schlaf zur Vermeidung von Schnarchgeräuschen besteht aus einem flachen Köper, der durch ein selbstklebendes Material, insbesondere ein Pflaster gebildet ist, und einen ersten Bereich (5), der dazu ausgebildet ist, im Bereich des Oberkiefers (105) am Gesicht eines Benutzers befestigt zu werden, einen zweiten Bereich (10), der dazu ausgebildet ist, im Bereich des Unterkiefers des Gesichts bzw. am Kinn (110) des Benutzers befestigt zu werden, sowie einen beide Bereiche miteinander verbindenden Verbbindungsbereich (15) aufweist.

## Beschreibung

Die vorliegende Erfindung betrifft eine Vorrichtung, mit deren Hilfe das Schnarchen vermieden werden soll.

Das Problem des Schnarchens im Schlaf ist weit verbreitet. Untersuchungen haben gezeigt, dass circa 20% der Erwachsenen hiervon betroffen sind, wobei sich die Häufigkeit mit zunehmendem Alter deutlich erhöht. So schnarchen etwa 60% aller Männer und 40% aller Frauen mit einem Alter über 60 Jahren. Die hierbei entstehenden Geräusche können dabei eine Lautstärke von bis zu 70 dB erreichen, was insbesondere in der Nacht bzw. bei einer ansonsten ruhigen Umgebung eine erhebliche Lärmbelästigung darstellt. Unabhängig davon allerdings stellt Schnarchen auch eine Gefahr für die Gesundheit dar.

Zwar ist Schnarchen in der Regel harmlos und lediglich für die Umgebung störend, in einigen Fällen kann es allerdings zu einer Blockade der Luftröhre kommen, was zu Atemaussetzern führt. Der Schlafende erhält dann zumindest vorrübergehend keine Luft mehr, wodurch er erwacht und erheblich in seinem Schlaf und der damit verbundenen Erholung gestört wird. Extrem lautes und unregelmäßiges Schnarchen kann dabei ein Hinweis auf die zuvor geschilderten Atempausen während des Schlafes sein, was auch als so genannte obstruktive Schlafapnoe bezeichnet wird. Diese Schlafapnoe ist eine potenziell lebensgefährliche Erkrankung, die bei 5% der Bevölkerung auftritt und überwiegend übergewichtige Menschen im mittleren Lebensalter betrifft. Da hier die Atmung gestört ist, erhalten die Patienten nicht ausreichend Sauerstoff, was letztendlich zu Tagesschläfrigkeit, Konzentrationsstörrungen und dergleichen führt und eine erhebliche Belastung für das Herz-Kreislaufsystem darstellt.

Zwar wurden bereits verschiedene Lösungen entwickelt, mit deren Hilfe das Entstehen von Schnarchgeräuschen vermieden werden soll, diese Lösungen sind allerdings wenig effektiv, meist verhältnismäßig teuer oder für den Benutzer in der Anwendung entsprechend unangenehm.

Der vorliegenden Erfindung liegt deshalb die Aufgabenstellung zugrunde, eine einfache aber effektive Lösung zur Unterdrückung bzw. Vermeidung von Schnarchgeräuschen anzugeben.

Die Aufgabe wird durch eine Anordnung, welche die Merkmale des Anspruchs 1 aufweist, gelöst. Vorteilhafte Weiterbildungen der Erfindung sind Gegenstand der abhängigen Ansprüche.

Die erfindungsgemäße Lösung beruht auf der Erkenntnis, dass Schnarchen dadurch entsteht, dass der Schlafende durch den geöffneten Mund atmet und die Luft hierbei in Schwingungen gerät. Die Gründe für eine Atmung durch den Mund während des Schlafs sind vielfältig. So kann beispielsweise die Nasenatmung durch Schnupfen, vergrößerte Mandeln oder durch eine Nasenscheidewand-Verkrümmung behindert sein. Auch ohne diese Einflüsse kann allerdings eine Atmung durch den Mund auftreten, da während des Schlafens die Backenmuskeln erschlaffen und den Unterkiefer herabsinken lassen. Grundsätzlich erschlafft während des Schlafs die gesamte Muskulatur, wobei hiervon auch der Rachen und Schlund betroffen sind. Die Zunge oder das so genannte Gaumensegel können hierbei die Atemwege versperren, weshalb der Schlafende durch den Mund einatmet, um besser Luft zu bekommen. Durch die Verengung oder den Verschluss der Atemwege beginnt allerdings das Gaumensegel, das Zäpfchen, die Rachenwand oder der Kehlkopfdeckel im Luftstrom während des Einatmens zu schwingen, was zu den bekannten und störenden Schnarchgeräuschen führt.

Die vorliegende Erfindung beruht nun auf dem Gedanken, das Entstehen von Schnarchgeräuschen dadurch zu verhindern, dass Bewegungen des menschlichen Unterkiefers während des Schlafs unterdrückt oder zumindest ausreichend stark reduziert werden. Dies wird erfindungsgemäße mit einer Anordnung erzielt, welche durch ein selbstklebendes Material, insbesondere ein Pflaster gebildet ist und einen ersten Bereich aufweist, der dazu ausgebildet ist, im Bereich des Oberkiefers am Gesicht eines Benutzers befestigt zu werden. Ein zweiter Bereich der erfindungsgemäßen Anordnung ist dazu ausgebildet, im Bereich des Unterkiefers am Gesicht des Benutzers befestigt zu werden, wobei beide Bereiche über einen Verbindungsbereich miteinander verbunden sind. Durch diese Lösung wird der Unterkiefer also trotz erschlaffender Muskulatur fixiert, sodass ein Herabsinken während des Schlafs verhindert wird. Dies wiederum führt dazu, dass die Schnarchgeräusche nicht auftreten können.

Erfindungsgemäß wird also eine Anordnung zur Unterdrückung oder Reduzierung von Bewegungen des menschlichen Unterkiefers zur Vermeidung von Schnarchgeräuschen vorgeschlagen, wobei die Anordnung aus einem flachen Körper aus selbstklebendem Material, insbesondere durch ein Pflaster gebildet ist, und einen ersten Bereich, der dazu ausgebildet ist, im Bereich des Oberkiefers am Gesicht eines Benutzers befestigt zu werden, sowie einen zweiten Bereich, der dazu ausgebildet ist, im Bereich des Unterkiefers am Gesicht des Benutzers befestigt zu werden, aufweist, und wobei beide Bereiche über einen Verbindungsbereich miteinander verbunden sind.

Die erfindungsgemäße Lösung zeichnet sich dadurch aus, dass sie sehr einfach anzuwenden ist und trotz allem eine zuverlässige Unterdrückung von Schnarchgeräuschen gewährleistet. Auch handelt es sich um eine äußerst kostengünstige Lösung, welche durch einen Benutzer als nicht störend empfunden wird. Dies stellt einen deutlichen Vorteil gegenüber bekannten so genannten Schnarchbinden dar, welche auf einem ähnlichen Prinzip beruhen und ein Absinken des Unterkiefers verhindern sollen. Es handelt sich hierbei allerdings um verhältnismäßig komplexe und dementsprechend teuer herzustellende Konstruktionen, welche um Kinn und Scheitel des Benutzers gebunden werden und teilweise auch als unangenehm empfunden werden.

Gemäß einer vorteilhaften Weiterbildung der vorliegenden Erfindung wird die Anwendung der erfindungsgemäßen Anordnung für den Benutzer weiter erleichtert bzw. angenehmer gestaltet, indem der Verbindungsbereich derart ausgestaltet ist, dass er den Mundbereich des Benutzers überspannt, wobei der Verbindungsbereich dann in diesem Abschnitt nicht-klebend ausgebildet ist. D.h., die Lippen werden durch die erfindungsgemäße Anordnung nicht fixiert und können nach wie vor leicht bewegt werden, was sich als sehr angenehm für den Benutzer herausgestellt hat. Der nicht-klebende Abschnitt kann hierbei in vorteilhafter Weise dadurch gebildet werden, dass der selbstklebende Körper an einer Seite vollständig klebend ausgebildet ist und einen umklappbaren oder umfaltbaren Bereich bzw. eine Lasche aufweist, der im umgeklappten bzw. umgefalteten Zustand dann den nicht-klebenden Abschnitt des Verbindungsbereichs bildet. Die erfindungsgemäße Anordnung ist also extrem einfach - vorzugsweise einstückig - aufgebaut und dementsprechend kostengünstig in ihrer Herstellung, trägt allerdings trotz allem zur zuverlässigen Unterdrückung von Schnarchgeräuschen bei.

Gemäß einer weiteren vorteilhaften Weiterbildung der Erfindung kann ferner vorgesehen sein, dass das selbstklebende Material insgesamt, zumindest jedoch der Verbindungsbereich in Verbindungsrichtung, also in Richtung von dem ersten zu dem zweiten Bereich hin eine geringfügige Flexibilität aufweist. Eine zumindest leichte Unterkieferbewegung wird hierdurch noch ermöglicht, was wiederum den Tragekomfort der erfindungsgemäßen Anordnung weiter erhöht. Trotzallem wird das Herabfallen des Unterkiefers zuverlässig verhindert.

Die konkrete Ausgestaltung der erfindungsgemäßen Anordnung kann unterschiedlich sein. Der Verbindungsbereich ist dabei allerdings vorzugsweise derart ausgestaltet, dass er - wie oben bereits erwähnt - den Mund des Benutzers überspannt, diesen allerdings nicht vollständig ab- bzw. überdeckt. Vorzugsweise wird dementsprechend der Verbindungsbereich durch einen oder mehrere schmale Verbindungsstege gebildet, welche den ersten, im Oberkieferbereich des Gesichts befestigten Bereich mit dem zweiten, den Unterkiefer fixierenden Bereich verbinden.

Nachfolgend soll die Erfindung anhand der beiliegenden Zeichnung näher erläutert werden. Es zeigen:
- Figur 1: eine Darstellung zur Verdeutlichung der Funktionsweise der erfindungsgemäßen Anordnung;
- Figuren 2a bis 2c: Ansichten eines ersten Ausführungsbeispiels einer erfindungsgemäßen Anordnung; und
- Figuren 3 bis 5: weitere denkbare Varianten einer erfindungsgemäßen Anordnung.

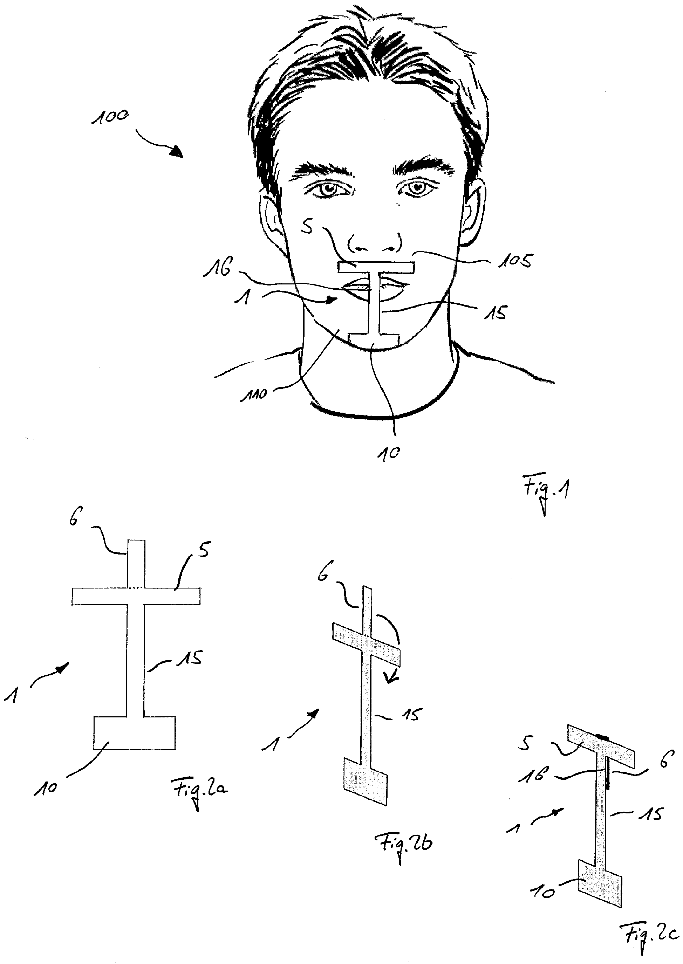

Anhand von Figur 1 soll zunächst die prinzipielle Funktionsweise der erfindungsgemäßen Anordnung erläutert werden. Gezeigt ist der Kopf 100 eines Benutzers, an dem die erfindungsgemäße Anordnung 1, die nachfolgend auch als Schnarchpflaster bezeichnet wird, angeordnet ist. Wie bereits erwähnt beruht die Funktion des erfindungsgemäßen Schnarchpflasters 1 darauf, dass ein Absinken des Unterkiefers 110 während des Schlafs verhindert wird. Dies wird mit Hilfe des erfindungsgemäßen Schnarchpflasters 1 dadurch erreicht, dass eine Verbindung zwischen dem Oberkieferbereich 105 bzw. allgemein einem festen Bereich des Gesichts mit dem Unterkiefer 110 bzw. dem Kinn erstellt wird, welche selbst bei einem Erschlaffen der Backenmuskulatur verhindert, dass der Unterkiefer 110 absinkt.

Das erfindungsgemäße Schnarchpflaster 1 besteht dementsprechend aus einem ersten Bereich 5, der oberhalb des Unterkiefers am Gesicht des Benutzers befestigt wird, sowie einem zweiten Bereich 10, der am Unterkiefer 10 fixiert wird. Mit Hilfe eines Verbindungsbereichs 15, der sich von dem ersten Bereich 5 zu dem zweiten Bereich 10 erstreckt, wird dann eine Bewegung des Unterkiefers 110, insbesondere dessen Herabfallen verhindert. Die Befestigung des ersten Bereichs 5 erfolgt hierbei vorzugsweise - wie dargestellt - im Oberlippenbereich des Benutzers, während hingegen der zweite Bereich 10 am Kinn des Benutzers angreift. Alternative Anordnungen wären ebenfalls denkbar, sofern - wie bereits erwähnt - der Unterkiefer 110 bezüglich des restlichen Schädels fixiert wird. Die dargestellte Anordnung hat sich allerdings als besonders angenehm für einen Benutzer herausgestellt.

Das Fixieren des erfindungsgemäßen Schnarchpflasters 1 am Gesicht des Benutzers erfolgt insbesondere dadurch, dass dieses aus einem klebenden Material besteht, bzw. an einer Flachseite selbstklebend ausgebildet ist. Dabei wäre es im Hinblick auf eine einfache Herstellung des Schnarchpflasters 1 wünschenswert, wenn die gesamte Flachseite in gleicher Weise, also klebend ausgebildet sein kann. Es ergibt sich hierbei allerdings das Problem, dass es von einem Benutzer des erfindungsgemäßen Schnarchpflasters 1 in der Regel als unangenehm empfunden wird, wenn der den Mund, insbesondere die Lippen überspannende Abschnitt 16 des Verbindungsbereichs 15 klebend ausgeführt ist. Mit Hilfe einer einfachen Maßnahme kann dies vermieden werden, wie nachfolgend anhand der Figuren 2a bis 2c erläutert wird.

Diese Figuren zeigen dabei eine erste vorteilhafte Ausführungsform des erfindungsgemäßen Schnarchpflasters 1, wobei Figur 2a zunächst eine Frontalansicht vor der Anwendung des Schnarchpflasters 1 zeigt. Erkennbar ist bereits die Struktur bestehend aus dem ersten Bereich 5, dem zweiten Bereich 10 und dem Verbindungsbereich 15. Wie erkennbar ist, ist allerdings ferner am oberen Ende des ersten Bereichs 5 noch eine nach oben gerichtete Lasche 6 vorgesehen, welche hinsichtlich ihrer Breite dem Verbindungsbereich 15 entspricht oder ggf. etwas größer bemessen ist. Vor der Anwendung, also vor dem Fixieren des Schnarchpflasters 1 am Gesicht des Benutzers wird nunmehr nach Entfernen einer nicht gezeigten, bei Pflastern üblichen Schutzabdeckung - wie in den Figuren 2b und 2c dargestellt - die Lasche 6 entlang der durch Punkte angedeuteten Faltlinie umgeklappt bzw. umgefaltet und über den oberen Abschnitt des Verbindungsbereichs 15 gelegt. Da lediglich die dem Gesicht des Benutzers zugewandte Seite des Schnarchpflasters 1 klebend ausgebildet ist, hat dies zur Folge, dass nunmehr im oberen Abschnitt 16 des Verbindungsbereichs 15 ein nicht-klebender Bereich gebildet ist, der den Mund bzw. die Lippen des Benutzers überspannt. Mit dieser einfachen Maßnahme wird also sichergestellt, dass das Schnarchpflaster 1 nicht auf den Lippen des Benutzers klebt, obwohl dieses einstückig ausgestaltet ist.

Es kann ferner vorgesehen sein, dass das Schnarchpflaster 1 entweder über die gesamte Fläche, zumindest jedoch im Verbindungsbereich 15 eine geringfügige Flexibilität aufweist. Hierdurch werden geringe Unterkieferbewegungen ermöglicht, was wiederum den Tragekomfort der erfindungsgemäßen Anordnung erhöht. Selbstverständlich kann auch der Benutzer selbst hierauf einen gewissen Einfluss nehmen, indem er den zweiten Abschnitt 10 derart an einer Stelle am Kinn befestigt, dass nach wie vor ein gewisser Spielraum für Unterkieferbewegungen verbleibt. Letztendlich ist allerdings dieser Spielraum derart bemessen, dass ein Vollständiges Herabsinken und damit das Entstehen von Schnarchgeräuschen nicht zugelassen wird.

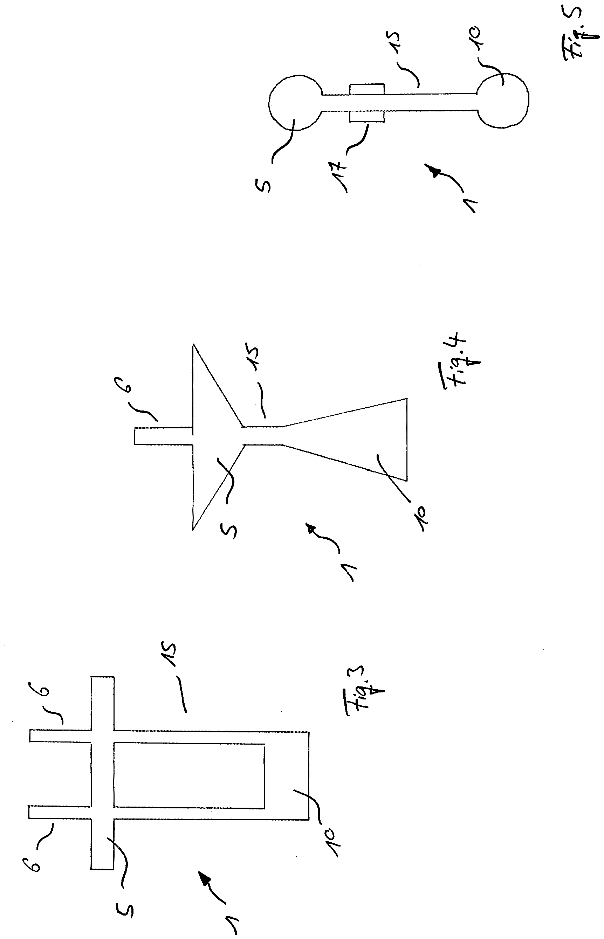

Hinsichtlich der genauen Ausgestaltung und Struktur des erfindungsgemäßen Schnarchpflasters 1 können unterschiedliche Varianten vorgesehen sein, solange der oben genannte Zweck erreicht wird, nämlich den Unterkiefer 110 bezüglich des Oberkiefers 105 des Benutzers zu fixieren. Die Figuren 3 bis 5 zeigen beispielhaft drei weitere denkbare Varianten, wobei gleiche Elemente des erfindungsgemäßen Schnarchpflasters 1 mit gleichen Bezugszeichen versehen sind.

Auch bei diesen drei Ausführungsbeispielen sind jeweils ein erster und ein zweiter Bereich 5 bzw. 10 vorgesehen, die am Ober- bzw. Unterkiefer zu befestigen und über einen Verbindungsbereich 15 miteinander verbunden sind. Dabei kann die Verbindung - wie in Figur 4 dargestellt - auch über mehrere parallel zueinander verlaufende Stege erfolgen, wobei dann jeweils den Stegen entsprechende Laschen 6 an der Oberseite des ersten Bereichs 5 vorgesehen sind, um den nicht-klebenden Abschnitt im Mundbereich des Benutzers zu erzielen. Die verschiedenen Varianten sind dabei hinsichtlich ihres Verbindungsbereichs 15 grundsätzlich derart ausgeführt, dass der Mund des Benutzers nicht vollständig abgedeckt bzw. überdeckt wird sondern grundsätzlich eine gewisse Öffnung verbleibt, durch welche ggf. auch ein Atmen ermöglicht wird.

Bei der Variante gemäß Figur 5 ist kein Umklappen einer Lasche vorgesehen, um einen nicht-klebenden Abschnitt zu bilden. Stattdessen ist hier im Verbindungsbereich 15 ein separates zweites Element 17 vorgesehen, welches mit dem Verbindungsabschnitt 15 verklebt ist und eine dem Mund zugewandte, nicht-klebende Auflagefläche bildet. Diese Ausführungsform zeichnet sich durch einen hohen Tragekomfort für den Benutzer aus, ist allerdings aufgrund der Tatsache, dass sie aus zwei miteinander verbundenen Elementen besteht, etwas aufwändiger in der Herstellung.

Letztendlich wird also durch die erfindungsgemäße Lösung eine Möglichkeit geschaffen, in einfacher aber effektiver Weise das Absinken des Unterkiefers im Schlaf zu verhindern. Schnarchgeräusche können hierdurch zuverlässig vermieden werden.

## Patentansprüche

1. Anordnung (1) zur Unterdrückung oder Reduzierung von Bewegungen des menschlichen Unterkiefers im Schlaf zur Vermeidung von Schnarchgeräuschen, bestehend aus einem flachen Köper, der durch ein selbstklebendes Material, insbesondere ein Pflaster gebildet ist, mit
• einem ersten Bereich (5), der dazu ausgebildet ist, im Bereich des Oberkiefers (105) am Gesicht eines Benutzers befestigt zu werden,
• einem zweiten Bereich (10), der dazu ausgebildet ist, im Bereich des Unterkiefers des Gesichts bzw. am Kinn (110) des Benutzers befestigt zu werden, sowie
• einen beide Bereiche miteinander verbindenden Verbbindungsbereich (15).

2. Anordnung nach Anspruch 1,
**dadurch gekennzeichnet,**
**dass** der Verbindungsbereich (15) den Mundbereich des Benutzers überspannt, wobei der Verbindungsbereich (15) einen dem Gesicht zugewandten nicht-klebenden Abschnitt (16) aufweist.

3. Anordnung nach Anspruch 2,
**dadurch gekennzeichnet,**
**dass** der flache Körper an einer Seite vollständig klebend ausgebildet ist und einen umklappbaren oder umfaltbaren Bereich (6) aufweist, der im umgeklappten bzw. umgefalteten Zustand den nicht-klebenden Abschnitt (16) des Verbindungsbereichs (15) bildet.

4. Anordnung nach Anspruch 3,
**dadurch gekennzeichnet,**
**dass** der umklappbare oder umfaltbare Bereich (6) hinsichtlich seiner Abmessung dem Verbindungsbereich (15) entspricht oder größer bemessen ist.

5. Anordnung nach einem der vorherigen Ansprüche,
**dadurch gekennzeichnet,**
**dass** der flache Körper einstückig ausgebildet ist.

6. Anordnung nach Anspruch 2,
**dadurch gekennzeichnet,**
**dass** der nicht-klebende Abschnitt (16) durch ein separates, mit dem Verbindungsabschnitt (15) auf der dem Gesicht zugewandten Seite verbundenes Abdeckelement (17) gebildet ist.

7. Anordnung nach einem der vorherigen Ansprüche,
**dadurch gekennzeichnet,**
**dass** zumindest der Verbindungsbereich (15) in Richtung von dem ersten zu dem zweiten Bereich (5, 10) eine geringfügige Flexibilität aufweist.

8. Anordnung nach einem der vorherigen Ansprüche,
**dadurch gekennzeichnet,**
**dass** der Verbindungsbereich (15) durch einen oder mehrere Verbindungsstege gebildet ist.
